# EUROPEAN PATENT APPLICATION

(11) **EP 1 870 452 A1**
(43) Date of publication of application: **26.12.2007**
(21) Application number: 06731342.9
(22) Date of filing: 31.03.2006
(51) Int. Cl.: C12N 5/06, A61K 35/12, A61P 37/06

(54) **PROCESS FOR PRODUCTION OF REGULATORY T CELL**

(30) Priority: 01.04.2005 JP 2005106887
(71) Applicant: Kyoto University, Kyoto-shi, Kyoto 606-8501 (JP)
(72) Inventor: KOSHIBA, Takaaki, Grad. School of Medic.Kyoto Unv., Kyoto-shi, Kyoto 606-8501 (JP); ITO, Atsushi, Graduate School of Med. Kyoto Unv, Kyoto-shi, Kyoto 606-8501 (JP); TANAKA, Koichi, Graduate School of Med.Kyoto Unv., Kyoto-shi, Kyoto 606-8501 (JP); SAKAGUCHI, Shimon, Inst.Frontier Medical Sc. Kyoto, Kyoto-shi, Kyoto 6068501 (JP)
(74) Representative: Duckworth, Timothy John
(86) International application number: PCT/JP2006/307394
(87) International publication number: WO 2006/107101

(57) **Abstract**

The present invention provides a method of producing a regulatory T cell, which includes culturing a peripheral CD25⁺CD4⁺ T cell of a naive phenotype to give the regulatory T cell. The CD25⁺CD4⁺ T cell can be cultured under stimulation with an antigen. According to the method, a mammalian regulatory T cell can be produced in vitro and an antigen-specific regulatory T cell can also be produced. The regulatory T cell produced by the method is useful as an immunomodulator for the prophylaxis or treatment of rejection reaction in organ transplantation, allergic disease, autoimmune disease, graft-versus-host disease (GVHD), infertility and the like.

## Description

### Technical Field

The present invention relates to a method of producing a regulatory T cell, a regulatory T cell produced by the method, an immunomodulator comprising the regulatory T cell as an active ingredient, a method of producing the immunomodulator, a kit for the production of a regulatory T cell and the like.

### Background Art

After organ transplantation, immunosuppressants are used to inhibit the rejection. However, immunosuppressants often induce infection, and the leading cause of the death of the patients after transplantation is infection. As evidenced above, in the current medical transplantation, while the surgical technique has been established, rejection suppressive measures and anti-infection measures produce conflicts in the treatment strategy. Accordingly, it is an issue of urgency to simultaneously achieve both of them. Given such situation, the study of the mechanism of immune tolerance - the state where a transplanted organ sufficiently functions without using an immunosuppressant - and the method of actively inducing the immune tolerance is becoming the main pillar in the field of medical transplantation. This means that the study of medical transplantation has completed the level of organ preservation and improvement of surgical techniques, and clearly entered the second stage.

In recent years, there are findings rapidly accumulated at the animal experiment level that CD25⁺CD4⁺ regulatory T cell (regulatory T cell(s) is sometimes referred to as Treg(s)) plays an important role in the immune tolerance after organ transplantation. The present inventors have confirmed that this cell grows in patients with naturally established immune tolerance after living liver transplantation (Am J Transpl, 4, 2118, 2004). The present inventors have also shown that a skin graft is not rejected in the mouse system by employing a method including once separating regulatory T cells, culturing the cells in vitro in a donor antigen-specific manner and returning them into the body, a so-called cell adoptive immunotherapy (Int Immunol., 16, 1189-1201, 2004). From these, a method effective for actively inducing the immune tolerance in medical transplantation in human is presumed to be in vitro proliferation of regulatory T cells and transferring them into the patient, namely, cell adoptive immunotherapy. When regulatory T cells are proliferated in vitro, maintenance of regulatory capacity thereof is indispensable for the cell adoptive immunotherapy (Nat Rev Immunol., Mar;3(3), 199-210, 2003). However, in vitro proliferation of human regulatory T cells in a high fold without losing the regulatory capacity thereof has not been reported.

Human CD25⁺CD4⁺ regulatory T cell has been reported to play an essential role in vivo in the immune tolerance to the endogenous autoantigen, and more recently, in the transplantation immune tolerance after organ transplantation (Annu Rev Immunol., 22, 531-562, 2004, Nat Rev Immunol., 2, 389-400, 2002, Nat Rev Immunol., 3, 199-210, 2003). In vivo, human Tregs are characterized by memory phenotype-CD45RO⁺ in addition to the property of the cell surface CD4⁺ and CD25^{high+}, Foxp3 expression, intracellular CTLA-4, cell surface GITR, TNFRII, and non-responsiveness/immunosuppressive ability to autoantigen and alloantigen and the like (J Immunol., 167, 1245-1253, 2001, J Exp Med., 193, 1285-1294, 2001, J Exp Med., 193, 1295-1302, 2001, Blood, 98, 2736-2744, 2001, Eur J Immunol., 32, 1621-1630, 2002, Int Immunol., 16, 1643-1656, 2004, Exp Hematol., 32, 622-629, 2004). In experiment, when thymus cells free of CD25⁺CD4⁺ cells are adoptively transferred into an immunodeficient host, autoimmune diseases are developed (J Immunol., 162, 5317-5326, 1999), and in a certain rodent model, isolation of thymus before transplantation prevents transplantation immune tolerance mediated by Tregs (Transplantation, 76, 588-596, 2003). In agreement with these experimental evidences, CD25⁺CD4⁺ T cell expressing Foxp3 at a high level are present in human thymus (Blood, 102, 4107-4114, 2003, Eur J Immunol, 35, 383-390, 2005). Based on these observations, Tregs seem to be derived from thymus, emigrate toward periphery, and contribute to the establishment of peripheral immune tolerance. Although CD25⁺CD4⁺ T cell in human thymus expresses Foxp3 and exhibits the feature of non-responsiveness/immunosuppressive ability, it has a naive cell phenotype, CD45RA, instead of CD45RO (Blood, 102, 4107-4114, 2003, Eur J Immunol., 35, 383-390, 2005, Eur J Immunol., 31, 1247-1254, 2001, J Exp Med, 196, 379-387, 2002, Immunology, 106, 190-199, 2002). This means that the peripheral Treg and intrathymic CD25⁺CD4⁺ T cell have different phenotypes. Accordingly, Treg with a naive phenotype derived from thymus should be developed from Treg with a memory phenotype. However, the differentiation cascade of peripheral Treg remains to be elucidated in both animal model and human. Thus, questions arise as to how and where the thymus-derived "naive type: immature Treg" acquires the properties of "memory type: mature Treg".

CD45RA⁺CD25⁺CD4⁺ T cell in peripheral blood have heretofore been considered different from Tregs. This is because this population does not exhibit non-responsiveness and suppressive ability, but rather, proliferates on stimulation with alloantigen and agonistic anti-CD3 antibody (Int Immunol., 16, 1643-1656, 2004). In periphery, instead, CD45RA⁻CD25⁺CD4⁺ T cell having the feature of non-responsiveness/immunosuppressive ability represent Tregs. However, the following points are interesting.
(1) CD45RA⁺CD25⁺CD4⁺ T cell in peripheral blood commonly have CD45RA⁺, a naive cell phenotype of CD25⁺CD4⁺ T cell in thymus or cord blood (Immunology, 106, 190-199, 2002, Exp Hematol., 32, 622-629, 2004).
(2) While several studies have analyzed the functional side of CD45RA⁺CD25⁺CD4⁺ T cell only at the time point of isolation from peripheral blood (Int Immunol., 16, 1643-1656, 2004, J Exp Med., 193, 1285-1294, 2001, Eur J Immunol., 32, 1621-1630, 2002), functional changes after cultivation of the cells in vitro and genetic characteristics have not been studied in detail as yet.

In view of the above-mentioned situation, the present invention aims at provision of a new method of producing a regulatory T cell in mammal, particularly a primate such as human and the like.

### Disclosure of the Invention

The present inventors have focused on the properties of CD45RA⁺CD25⁺CD4⁺ T cell in peripheral blood and conducted intensive studies in an attempt to achieve the above-mentioned object, and found the following.

To be specific, CD45RA⁺CD25⁺CD4⁺ T cell have many naive phenotypes, and the frequency gradually decreased in an age-dependent manner. Despite the different phenotypes from peripheral Tregs, this population expressed Foxp3 at a very high level, and expressed, like Tregs, CLTA-4 in the cell and TNFRII on the cell surface. At the time point of separation from peripheral blood, unlike Tregs, this population did not exhibit the features of non-responsiveness and immunosuppressive ability, but rather, proliferated against an alloantigen in the presence of an agonistic anti-CD3 antibody. Importantly, the population acquired the features of non-responsiveness/antigen-specific immunosuppressive ability, possessed by Tregs, after cultivation and proliferation with the alloantigen.

Based on these observation results, the present inventors completed the present invention. Accordingly, the present invention relates to the following.
[1] A method of producing a regulatory T cell, which comprises culturing a peripheral CD25⁺CD4⁺ T cell of a naive phenotype to give the regulatory T cell.
[2] The method of the above-mentioned [1], wherein the naive phenotype is at least one phenotype selected from the group consisting of CD45RA⁺, CD45RO^{low+}, CD45RB^{high+}, CD62L^{high+} and CD38⁺.
[3] The method of the above-mentioned [1], wherein the CD25⁺CD4⁺ T cell has at least one phenotype selected from the group consisting of Foxp3⁺, intracellular CTLA-4⁺ and cell surface TNFRII⁺.
[4] The method of the above-mentioned [1], wherein the CD25⁺CD4⁺ T cell is derived from primate.
[5] The method of the above-mentioned [1], wherein the CD25⁺CD4⁺ T cell is cultured in the presence of an antigen.
[6] The method of the above-mentioned [5], wherein the antigen is an immunologically non-autologous cell.
[7] The method of the above-mentioned [6], wherein the immunologically non-autologous cell is an allogenic cell.
[8] The method of the above-mentioned [5], wherein the obtained regulatory T cell is specific for the antigen.
[9] The method of the above-mentioned [1], wherein the CD25⁺CD4⁺ T cell is cultured in the presence of a T cell growth factor.
[10] The method of the above-mentioned [1], wherein the regulatory T cell has at least one phenotype selected from the group consisting of CD45RO^{mid+,} CD45RB^{high+}, CD62L^{low+} and GITR⁺.
[11] A regulatory T cell produced by the method of any one of the above-mentioned [1] to [10].
[12] A CD25⁺CD4⁺ regulatory T cell having at least one phenotype selected from the group consisting of CD45RO^{mid+,} CD45RB^{high+}, CD62L^{low+} and GITR⁺.
[13] An immunomodulator comprising the regulatory T cell of the above-mentioned [11] or [12] as an active ingredient.
[14] The agent of the above-mentioned [13], which is used for the introduction of immune tolerance.
[15] A method of producing an immunomodulator, which comprises (1) a step of culturing a peripheral CD25⁺CD4⁺ T cell of a naive phenotype to give a regulatory T cell; (2) a step of mixing the regulatory T cell with a pharmaceutically acceptable carrier to give the immunomodulator.
[16] A kit for the production of a regulatory T cell, which comprises a reagent for providing a peripheral CD25⁺CD4⁺ T cell of a naive phenotype.
[17] Use of the regulatory T cell of the above-mentioned [11] or [12] for the production of an immunomodulator.
[18] The use of the above-mentioned [17], wherein the immunomodulator is an immune tolerance-inducing agent.
[19] A method of regulating immunity, which uses the regulatory T cell of the above-mentioned [11] or [12].
[20] The method of the above-mentioned [19], which is a method of inducing immune tolerance.
[21] A commercial package comprising an immunomodulator composition comprising the regulatory T cell of the above-mentioned [11] or [12] and a pharmaceutically acceptable carrier, and a written matter stating that the composition can or should be used for immune regulation.
[22] The package of the above-mentioned [21], wherein the immune regulation is induction of immune tolerance.

Using the method of the present invention, a mammalian regulatory T cell can be efficiently produced in vitro, and an antigen-specific regulatory T cell can also be produced. The regulatory T cell produced by the method can be used as an immunomodulator for the prophylaxis or treatment of the rejection in organ transplantation, an allergic disease, an autoimmune disease, a graft-versus-host disease (GVHD), infertility and the like.

### Brief Description of the Drawings

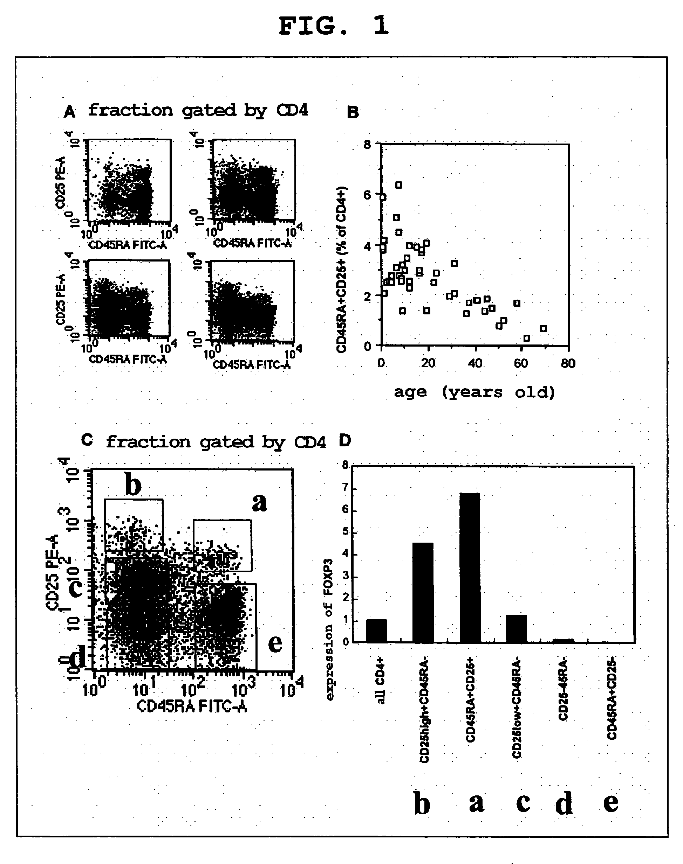

Fig. 1 shows the analysis results of CD45PA⁺CD25⁺CD4⁺ T cell. (A) shows FACS profiles of CD45RA⁺CD25⁺CD4⁺ T cell fractions in different generations wherein a representative FACS profile of each CD45RA⁺CD25⁺CD4⁺ T cell fraction is shown (4 months old: upper left, 10 years old: upper right, 22 years old: lower left, and 35 years old: lower right). (B) is a graph showing the correlation between age (X axis) and the proportion of CD45RA⁺CD25⁺CD4⁺ T cell (Y axis). (C) shows the definitions of (a) CD45RA⁺CD25⁺CD4⁺ T cell, (b) CD45RA⁻ CD25^{high+}CD4⁺ T cell, (c) CD45RA⁻CD25^{low+}CD4⁺ T cell, (d) CD45RA⁻ CD25⁻CD4⁺ T cell and (e) CD45RA⁺CD25⁻CD4⁺ T cell. (D) is a graph showing the FOXP3 expression level in each cell fraction.

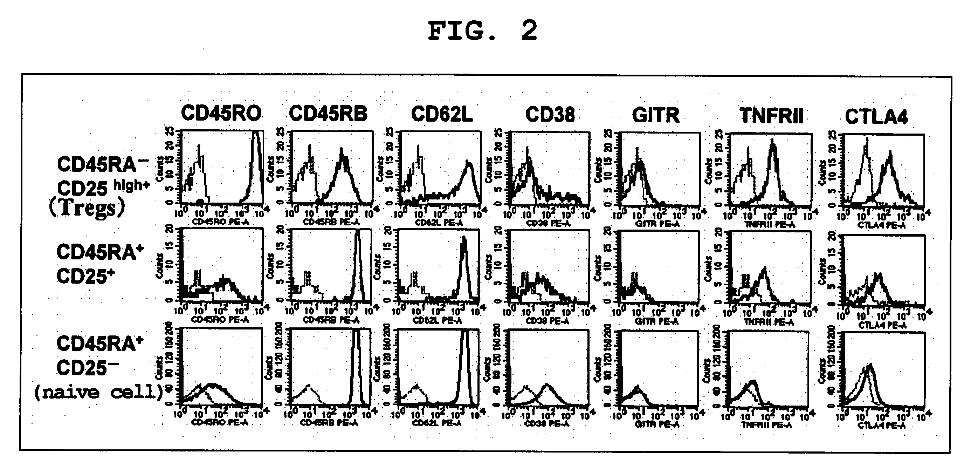

Fig. 2 shows the histogram analysis of naive/memory and Treg-specific phenotype of CD45RA⁺CD25⁺CD4⁺, CD45RA⁻CD25^{high+}CD4⁺ and CD45RA⁺CD25⁻CD4⁺ cell fractions. The analysis results using specific antibodies against the indicated antigens are shown with a solid line. The isotype control for each phenotype is shown with a dotted line.

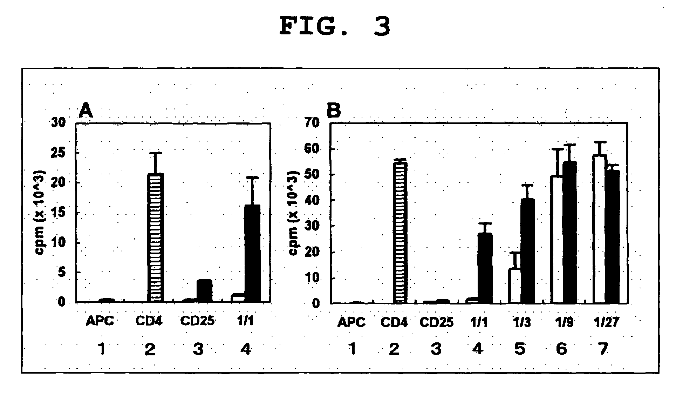

Fig. 3 shows comparison of functional properties of CD45RA⁺CD25⁺CD4⁺ T cell fraction and CD45RA⁻CD25^{high+}CD4⁺ T cell fraction at the time point of isolation from peripheral blood. The vertical axis shows [³H] thymidine uptake (cpm (x 10^3)), the black columns show CD45RA⁺CD25⁺CD4⁺ T cell, and white columns show CD45RA⁻CD25^{high+}CD4⁺ T cell. (A) shows the effect of CD45RA⁺CD25⁺CD4⁺ T cell and CD45RA⁻CD25^{high+}CD4⁺ T cell on the proliferation of all-CD4⁺ T cell in the presence of an agonistic anti-CD3 antibody. Lane 1 (APC): allogenic PBMC alone, lane 2 (CD4): allogenic PBMC + all CD4⁺ T cell, lane 3 (CD25): allogenic PBMC + CD45RA⁺CD25⁺CD4⁺ T cell or CD45RA⁻ CD25^{high+}CD4⁺ T cell, lane 4 (1/1): allogenic PBMC + all CD4⁺ T cell + CD45RA⁺CD25⁺CD4⁺ T cell or CD45RA⁻CD25^{high+}CD4⁺ T cell. (B) shows the effect of CD45RA⁺CD25⁺CD4⁺ T cell and CD45RA⁻ CD25^{high+}CD4⁺ T cell on the proliferation of all CD4⁺ T cell in the absence of an agonistic anti-CD3 antibody. Lane 1 (APC): allogenic PBMC alone, lane 2 (CD4): allogenic PBMC + all CD4⁺ T cell, lane 3 (CD25): allogenic PBMC + CD45RA⁺CD25⁺CD4⁺ T cell or CD45RA⁻CD25^{high+}CD4⁺ T cell, lanes 4 - 7: allogenic PBMC + all CD4⁺ T cell + CD45RA⁺CD25⁺CD4⁺ T cell or CD45RA⁻CD25^{high+}CD4⁺ T cell (CD45RA⁺CD25⁺CD4⁺ T cell or CD45RA⁻CD25^{high+}CD4⁺ T cell are added in the number of the indicated proportion (proportion relative to the number of all CD4⁺ T cell)).

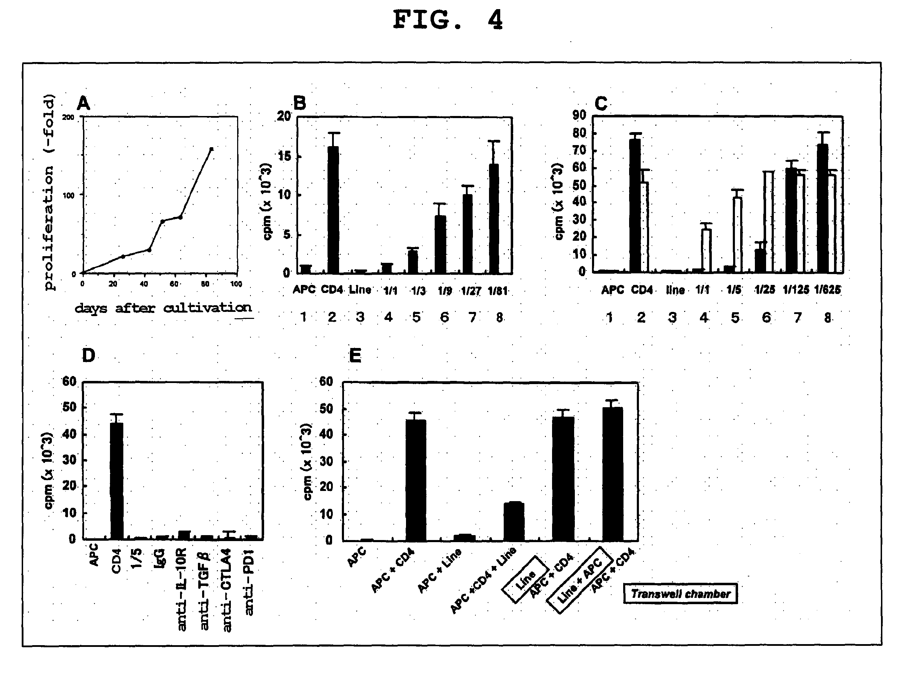

Fig. 4 shows the analysis results of functional properties of CD45RA⁺CD25⁺CD4⁺ T cell line. (A) shows proliferation of CD45RA⁺CD25⁺CD4⁺ T cell line. The vertical axis shows the number of cells as a relative value when the number of cells at the start of the cultivation is 1, and the transverse axis shows the number of days after the start of the cultivation. (B) shows the effect of CD45RA⁺CD25⁺CD4⁺ T cell line on the proliferation of all CD4⁺ T cell in the presence of an agonistic anti-CD3 antibody. Lane 1 (APC): allogenic PBMC alone, lane 2 (CD4): allogenic PBMC + all CD4⁺ T cell, lane 3 (Line): allogenic PBMC + CD45RA⁺CD25⁺CD4⁺ T cell line, lanes 4 - 8: allogenic PBMC + all CD4⁺ T cell + CD45RA⁺CD25⁺CD4⁺ T cell line (CD45RA⁺CD25⁺CD4⁺ T cell lines are added in the number of the indicated proportion (proportion relative to the number of all CD4⁺ T cell)) (C) shows antigen-specific suppressive property of CD45RA⁺CD25⁺CD4⁺ T cell line. Lane 1 (APC): allogenic PBMC alone, lane 2 (CD4): allogenic PBMC + all CD4⁺ T cell, lane 3 (Line): allogenic PBMC + CD45RA⁺CD25⁺CD4⁺ T cell line, lanes 4 - 8: allogenic PBMC + all CD4⁺ T cell + CD45RA⁺CD25⁺CD4⁺ T cell line (CD45RA⁺CD25⁺CD4⁺ T cell lines are added in the number of the indicated proportion (proportion relative to the number of all CD4⁺ T cell)). Black column shows the same allogenic PBMC as allogenic PBMC used for the proliferative cultivation, and white column shows allogenic PBMC different from allogenic PBMC used for the proliferative cultivation (outsider PBMC). (D) shows the effect of an antibody against IL-10R, TGF-β, CTLA-4 or PD-1 on the suppressive property of CD45RA⁺CD25⁺CD4⁺ T cell line. (E) shows the effect of cell-cell contact avoidance on the suppressive property of CD45RA⁺CD25⁺CD4⁺ T cell line. In the Figure, APC shows allogenic PBMC, CD4 shows all CD4⁺ T cell, and Line shows CD45RA⁺CD25⁺CD4⁺ T cell line. The cells enclosed in a rectangle are placed in the upper compartment of the transwell chamber.

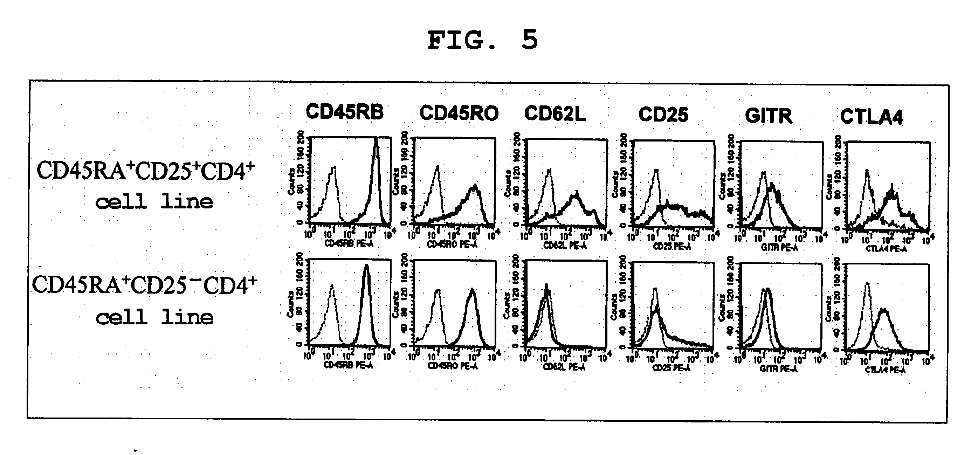

Fig. 5 shows histogram analysis of Tregs-specific phenotype of CD45RA⁺CD25⁺CD4⁺ T cell line and CD45RA⁺CD25⁻CD4⁺ T cell line. The analysis results using specific antibodies against the indicated antigens are shown with a solid line. The isotype control for each phenotype is shown with a dotted line.

### Detailed Description of the Invention

The present invention provides a method of producing a regulatory T cell, which comprises culturing peripheral CD25⁺CD4⁺ T cell of a naive phenotype to give the regulatory T cell.

The regulatory T cell means a T cell having an ability (immunosuppressive ability) to inhibit activation (proliferation, production of cytokine etc.) of reactive T cell (e.g., all CD4⁺ T cell, CD25⁻CD4⁺ T cell etc.) when it is cultivated with the reactive T cell under the conditions allowing cell contact and subjected to T cell receptor-mediated stimulation. The regulatory T cell is generally present in a CD25⁺CD4⁺ T cell fraction. The regulatory T cell itself does not show a substantial proliferative reaction on T cell receptor-mediated stimulation, and can be non-responsive.

In the method of the present invention, a peripheral CD25⁺CD4⁺ T cell of a naive phenotype is provided first. The cell can be derived from a mammal. While the mammal is not particularly limited as long as it can produce a regulatory T cell according to the method of the present invention, for example, experiment animals such as rodents (e.g., mouse, rat, hamster, guinea pig and the like), rabbit and the like; domestic animals such as swine, bovine, goat, horse, sheep, mink and the like; pets such as dog, cat and the like; and primates such as human, monkey, rhesus monkey, marmoset, orangutan, chimpanzee, cynomolgus monkey and the like can be mentioned. The mammal is preferably a primate, more preferably human.

The CD25⁺CD4⁺ T cell to be applied to the method of the present invention is "peripheral" CD25⁺CD4⁺ T cell. The "peripheral" means "extrathymic" and "other than cord blood". Accordingly, the CD25⁺CD4⁺ T cell to be applied to the present invention is not particularly limited as long as it is present in a peripheral tissue (tissue other than thymus and cord blood: e.g., peripheral blood, spleen, lymph node, intestine, liver etc.). In consideration of easy preparation, a cell in peripheral blood or spleen can be used.

The CD25⁺CD4⁺ T cell to be applied to the method of the present invention is of a naive phenotype. The "naive phenotype" refers to a phenotype of a T cell (naive T cell), which emigrates from thymus and is free of stimulation with an antigen. For example, the naive phenotype of human cell includes, but is not limited to, phenotypes such as CD45RA-positive (CD45RA⁺), CD45RO-low positive or CD45RO-negative (CD45RO^{low+} or CD45RO⁻), CD45RB-highly positive (CD45RB^{high+}), CD62L-highly positive (CD62L^{high+}), CD38-positive (CD38⁺) and the like. For example, in the case of human, the CD25⁺CD4⁺ T cell to be applied to the method of the present invention can preferably be of at least one naive phenotype, more preferably all naive phenotypes, selected from the group consisting of CD45RA⁺, CD45RO^{low+}, CD45RB^{high+}, CD62L^{high+} and CD38⁺. Even when the CD25⁺CD4⁺ T cell is derived from a mammal other than human, the phenotype may be the same as that of human. However, when the phenotype is defined by a marker molecule not inherently possessed by the animal species, interspecies difference may be taken into consideration, such as exclusion of the phenotype from the analysis and the like.

In the present specification, when the phenotype of a cell is represented by the presence or absence or the level of marker molecule (antigen) expression, unless otherwise specified, the phenotype is indicated by the presence or absence or the level of specific bond between an antibody and the marker molecule. The phenotype of a cell is generally determined by the presence or absence or the level of marker molecule expression by flow cytometric analysis and the like using an antibody specific to the marker molecule and the like. The "positive" expression of a marker molecule means that the marker molecule is expressed on the cell surface (or in the cell), and that a specific bond of an antibody against the marker molecule can be confirmed. Of these, the "highly positive" means that the level of expression of a marker molecule is relatively high, a cell population with a high level of expression of a marker molecule is present in a relatively large number, the rate of cell populations expressing a marker molecule is relatively large, and the like, as compared to other cells (or cell population) to be a control for comparison. The "low positive" means that the level of expression of a marker molecule is relatively low, cell populations with a low level of expression of a marker molecule is present in a relatively large number, the rate of cell populations expressing a marker molecule is relatively small, and the like, as compared to other cells (or cell population) to be a control for comparison.

When CD45RO-low positive (CD45RO^{low+}), CD45RB-highly positive (CD45RB^{high+}) or CD62L-highly positive (CD62L^{high+}) is indicated as a naive phenotype, it means that memory T cell ( T cell that emigrated from thymus, was subjected to stimulation with an antigen and still alive) is a control for comparison. As the memory T cell to be the control for comparison, CD45RA⁻CD25^{high+}CD4⁺ T cell (e.g., T cell contained in the region gated by b in Fig. 1C, etc.) and the like can be mentioned.

The peripheral CD25⁺CD4⁺ T cell of a naive phenotype, which is to be applied to the method of the present invention, can be of at least one phenotype selected from the group consisting of Foxp3-positive (Foxp3⁺), intracellular CTLA-4 positive (CTLA-4⁺) and cell surface TNFRII⁺. These phenotypes can be the phenotype of the aforementioned regulatory T cell (e.g., CD45RA⁻CD25^{high+}CD4⁺ T cell etc.). Of these, the CD25⁺CD4⁺ T cell to be used for the method of the present invention may exhibit the intensity of Foxp3 expression of an equivalent (about 0.5 - 2.0 times) or above expression level as compared to that of CD45RA⁻CD25^{high+}CD4⁺ T cell, based on the expression level of mRNA. The phenotype can be analyzed using Foxp3, generally by measuring the expression level of mRNA by RT-PCR and the like.

The peripheral CD25⁺CD4⁺ T cell of a naive phenotype, which is to be applied to the method of the present invention, can be of a phenotype of regulatory T cell, as mentioned above. However, as shown in the below-mentioned Examples and the like, it does not have functional properties of regulatory T cell, i.e., non-responsiveness and immunosuppressive ability (or the properties are extremely weak), but rather, proliferates on a stimulation with an antigen in the presence of an agonistic anti-CD3 antibody. On this point, a peripheral CD25⁺CD4⁺ T cell of a naive phenotype has different properties than those reported so far for a CD25⁺CD4⁺ T cell of a naive phenotype in thymus or cord blood.

The peripheral CD25⁺CD4⁺ T cell of a naive phenotype to be applied to the method of the present invention is preferably after isolation and purification. The cell can be isolated and purified from the aforementioned mammalian peripheral tissue (tissue other than thymus and cord blood) by a method known per se based on the aforementioned phenotype and the like.

For example, when a human peripheral CD25⁺CD4⁺ T cell of a naive phenotype is isolated and purified, a mononuclear cell fraction is prepared first from a peripheral tissue (e.g., peripheral blood etc.). The mononuclear cell fraction is prepared, for example, by density gradient centrifugation using Ficoll-Hypaque (Amersham Biosciences-Uppsala) and the like, apheresis and the like. Then, the mononuclear cell fraction is stained with an antibody labeled with a fluorescence dye, magnetic beads or the like, which is specific to an antigen (CD25, CD4, the aforementioned naive phenotype marker antigen (CD45RA etc.) etc.) specifically expressed on the cell surface of the intended cell, and the intended fraction is isolated and purified using a cell sorter, a magnetic column or the like. The cell sorter is preferably used to achieve a high level of purification.

Next, the provided peripheral CD25⁺CD4⁺ T cell of a naive phenotype is cultured.

For cell cultivation, the cultivation conditions generally used for lymphocyte cultivation technique can be used. For example, the cultivation temperature is generally within the range of about 30 - 40°C, preferably about 37°C. The CO₂ concentration is generally within the range of about 1 - 10%, preferably about 5%. The humidity is generally within the range of about 70 - 100%, preferably about 95 - 100%.

As the basal medium of a medium used for cultivation in the method of the present invention, a medium known per se can be used and is not particularly limited as long as a regulatory T cell can be produced by the method of the present invention. For example, DMEM, EMEM, RPMI-1640, α-MEM, F-12, F-10, M-199, HAM and the like can be mentioned. A modified medium for lymphocyte cultivation and the like can also be used, and a mixture of the above-mentioned basal media can also be used.

The medium may contain an additive known per se. While the additive is not particularly limited as long as a regulatory T cell can be produced by the method of the present invention, for example, an organic acid (e.g., sodium pyruvate etc.), an amino acid (e.g., L-glutamine etc.), a reducing agent (e.g., 2-mercaptoethanol etc.), a buffer (e.g., HEPES etc.), an antibiotic (e.g., streptomycin, penicillin, gentamicin etc.) and the like can be mentioned. Each of these additives is preferably contained within the concentration range known per se.

The medium may also contain a serum. The serum is not particularly limited as long as it is derived from mammal and a regulatory T cell can be produced by the method of the present invention. It is preferably a serum derived from the above-mentioned mammal (e.g., bovine fetal serum, human serum etc.). An alternative additive of serum (e.g., Knockout Serum Replacement (KSR) (manufactured by Invitrogen) etc.) can also be used. While the concentration of the serum is not particularly limited as long as a regulatory T cells can be produced by the method of the present invention, it is generally within the range of 0.1 - 30 (v/v) %.

To improve the production efficiency of a regulatory T cell according to the method of the present invention, a peripheral CD25⁺CD4⁺ T cell of a naive phenotype can be cultured in the presence of a T cell growth factor. While the T cell growth factor is not particularly limited as long as a regulatory T cell can be produced by the method of the present invention, for example, IL-2, IFN-γ and the like can be mentioned. The concentration of the T cell growth factor to be added to the medium is not particularly limited as long as a regulatory T cell can be produced by the method of the present invention. For example, when IL-2 is used, the concentration is generally about 0.1 - 10000 U/ml, for example, 1 - 5000 U/ml, preferably 10 - 2500 U/ml.

In the method of the present invention, a peripheral CD25⁺CD4⁺ T cell of a naive phenotype can be cultured in the presence of an antigen. A regulatory T cell specific to the antigen can be produced by cultivation in the presence of the antigen.

The antigen comprehensively means a substance that can be recognized by an antigen receptor on a cultured cell (e.g., T cell receptor) and can stimulate the cell via the receptor. The antigen includes, for example, not only an antigenic molecule such as peptide, protein, lipid, glycolipid and the like, but also an antigen mimic such as immunologically non-autologous cell, an agonistic antibody (e.g., OKT-3, which is anti-human CD3 antibody etc.), which recognizes a constituent molecule of an antigen receptor (CD3, TCRβ, TCRα etc.) or a costimulatory molecule (CD28 etc.), superantigen and the like. The immunologically non-autologous cell refers to a cell other than syngenic cell, and allogenic cell and xenogenic cell can be mentioned. The immunologically non-autologous cell is preferably an allogenic cell.

As the antigen to be used for stimulation with an antigen, one desirable for the object can be selected.

For example, when a regulatory T cell specific to a particular immunologically non-autologous cell is to be produced, a peripheral CD25⁺CD4⁺ T cell of a naive phenotype is cultured in the presence of the immunologically non-autologous cell. The immunologically non-autologous cell is preferably inactivated by a method known per se, for example, radiation (gamma-ray etc.), a treatment with an anticancer agent (mitomycin C etc.) and the like. The kind of the immunologically non-autologous cell is not particularly limited, and a cell derived from a desired tissue (e.g., peripheral blood mononuclear cell (PBMC) etc.) can be used. When a regulatory T cell for a recipient specific to an allogenic donor-derived cell is to be produced before transplantation from the donor, for example, a recipient-derived peripheral CD25⁺CD4⁺ T cell of a naive phenotype is cultured in the presence of the donor-derived cell. In this case, the donor-derived cell may be derived from the organ to be transplanted, or from a different tissue.

When a regulatory T cell specific to a particular antigen molecule (peptide, protein, lipid, glycolipid etc.) is to be produced, a peripheral CD25⁺CD4⁺ T cell of a naive phenotype is cultured in the presence of the antigen molecule. The antigen molecule includes, for example, cell- or tissue-derived antigen such as histocompatibility antigen and the like, a causative antigen of an allergic disease or a causative antigen of an autoimmune disease (a food-derived antigen, a pharmaceutical agent expected to exhibit antigenicity or a substance combined in a preparation, or an artificial organ-associated substance, or a denatured substance thereof (e.g., a thermally-denatured substance etc.)) and the like can be mentioned. As the histocompatibility antigen, major histocompatibility antigen (MHC antigen) and non-major histocompatibility antigen can be mentioned. The causative substance of allergy includes environmental or pollen antigen, fungal antigen, food antigen, artificial antigen and the like. For example, as the environmental or pollen antigen, mite, house dust, Japanese cedar pollen, ragweed and the like can be mentioned. As the fungal antigen, Candida, Alternaria, Aspergillus, cladosporium, Penicillium and the like can be mentioned. As the food antigen, albumen, milk, soybean, flour, buckwheat flour, mackerel, sardine, Japanese horse mackerel, shrimp, crab, pork, beef, chicken and the like can be mentioned. As the artificial antigen, a pharmaceutical agent, an artificial organ and the like can be mentioned. As the causative substance of autoimmune disease, a corresponding antigen to an autoantibody causing the disease, and the like can be mentioned.

In this case, the CD25⁺CD4⁺ T cell may be cultured in the presence of an antigen-presenting cell in order to certainly accomplish antigen presentation to the cell. While the antigen-presenting cell is not particularly limited as long as a regulatory T cell can be produced by the method of the present invention, a antigen-presenting cell syngenic with the peripheral CD25⁺CD4⁺ T cell of a naive phenotype to be cultivated (e.g., antigen-presenting cell obtained from the individual from which the CD25⁺CD4⁺ T cell is derived) is generally used. While the kind of the antigen-presenting cell is not particularly limited as long as it has an antigen-presenting ability and can produce a regulatory T cell by the method of the present invention, for example, PBMC, dendritic cell and the like can be used. The antigen-presenting cell is preferably inactivated by a method known per se, for example, radiation (gamma-ray etc.), a treatment with an anticancer agent (mitomycin C etc.) and the like.

In addition, when the repertoire variety of the antigen receptors possessed by the above-mentioned CD25⁺CD4⁺ T cell population to be cultivated is to be maintained to produce a regulatory T cell population reflecting the variety, the CD25⁺CD4⁺ T cell is cultured under a stimulation with an antigen mimic such as an agonistic antibody (e.g., OKT-3, which is an anti-human CD3 antibody etc.), which recognizes a constituent molecule of an antigen receptor (CD3, TCRβ, TCRα etc.), an agonistic antibody (e.g., anti-human CD28 antibody), which recognizes a costimulatory molecule (CD28 etc.), a superantigen and the like (Blood, 104, p. 895-903, 2004, Blood, 104, p. 453-61, 2004). Plural kinds of the antigen mimic can be used in combination and, for example, a combination of an agonistic antibody recognizing CD3 and an agonistic antibody recognizing CD28 and the like can be used. Using the antigen mimic, regulatory T cell populations having variety can be produced.

In addition, plural kinds of antigens can also be used in combination and, for example, a combination of an immunologically non-autologous cell and an agonistic antibody recognizing a constituent molecule of an antigen receptor (combination of allogenic cell and anti-CD3 antibody etc.) can be used.

By cultivation as mentioned above, peripheral CD25⁺CD4⁺ T cells of a naive phenotype in a culture are proliferated, and can be proliferated to 100-fold or more in number over a long term of 80 days or more.

As a result of the cultivation, a regulatory T cell can be obtained in the culture.

More specifically, as shown in the below-mentioned Examples, the cells that can be obtained by the method of the present invention acquire the ability to inhibit activation of reactive T cell (immunosuppressive ability) when they are cultured with reactive T cell under the conditions allowing cell contact and subjected to a T cell receptor-mediated stimulation. Furthermore, the obtained cell can also have the feature of non-responsiveness.

Importantly, when a peripheral CD25⁺CD4⁺ T cell of a naive phenotype is cultured in the presence of an antigen according to the method of the present invention, the obtainable regulatory T cell can be specific to the antigen used for the cultivation.

Whether or not the obtained cell is functional as a regulatory T cell can be confirmed. For example, the obtained cell is cultured with a reactive T cell (e.g., all-CD4⁺ T cell) under the conditions allowing cell contact and stimulated with an antigen, after which activation of the reactive T cell is determined. As the index of activation of reactive T cell, cell proliferation (e.g., [³H]-thymidine uptake) or production of cytokine (IL-2, IL-4, IFNγ etc.) is generally used. When activation of reactive T cell is inhibited as a result of the test, the obtained cell can be concluded to be functional as a regulatory T cell. Using, as an antigen to be used for stimulation, a combination of the same antigen as the antigen used in the production process of regulatory T cell and an antigen different from the antigen, whether or not the produced regulatory T cell is specific to the antigen can be confirmed.

While the regulatory T cell to be produced by the method of the present invention is of a CD25⁺CD4⁺ phenotype, the phenotype may be different from that of a naturally-occurring regulatory T cell in the periphery (e.g., human CD45RA⁻ CD25^{high+}CD4⁺ T cell etc.). Accordingly, a regulatory T cell to be produced by the method of the present invention can express CD45RO, which is in an intermediate level between peripheral CD25⁺CD4⁺ T cell of a naive phenotype and naturally-occurring regulatory T cell in the periphery (i.e., CD45RO^{mid+}). For example, when the expression level of cell surface CD45RO is compared using a fluorescence-labeled anti-CD45RO antibody in a flow cytometric analysis, the CD45RO expression level of human regulatory T cell to be produced by the method of the present invention is generally about 2- to 10-fold (e.g., about 5- to 8-fold) as compared to peripheral CD25⁺CD4⁺ T cell of a naive phenotype, and generally about 1/20- to 1/2-fold (e.g., about 1/6- to 1/3-fold) as compared to CD45RA⁻ CD25^{high+}CD4⁺ T cell. The expression level is compared by a flow cytometric analysis based on the comparison of the expression intensity at the peaks in a histogram in a graph wherein the vertical axis shows the number of cells, and the transverse axis shows the intensity of expression (fluorescence intensity).

The regulatory T cell to be produced by the method of the present invention expresses CD62L more weakly (CD62L-low positive (CD62L^{low+}) as compared to CD45RA⁻CD25^{high+}CD4⁺ T cell: expression level is generally about 1/3- to 1/30-fold of CD45RA⁻CD25^{high+}CD4⁺ T cell), and can be of a phenotype of GITR positive (GITR⁺).

Regulatory T cell to be produced by the method of the present invention expresses CD45RB more strongly (CD45RB-highly positive (CD45RB^{high+})) as compared to CD45RA⁻CD25^{high+}CD4⁺ T cell, and the expression level may be generally about 1.5-to 8-fold (e.g., 2.5- to 4.5-fold) of CD45RA⁻CD25^{high+}CD4⁺ T cell.

Accordingly, the present invention provides CD25⁺CD4⁺ regulatory T cell of at least one phenotype selected from the group consisting of CD45RO^{mid+}, CD45RB^{high+}, CD62L^{low+} and GITR⁺.

The regulatory T cell to be produced by the method of the present invention has a strong immunosuppressive ability. Therefore, when an immunoreaction is abnormally promoted in vivo for some reason, when an undesirable immunoreaction is ongoing in vivo, or when an undesirable immune response is predicted to occur in the future, or the like, the regulatory T cell is administered to a patient, whereby, in the body of patient, an abnormally-promoted immunoreaction can be suppressed, an undesirable immunoreaction can be suppressed, or an undesirable immunoreaction can be avoided.

For example, prior to organ transplantation from an allogenic donor, a recipient-derived peripheral CD25⁺CD4⁺ T cell of a naive phenotype is cultured in the presence of a donor-derived cell to give a regulatory T cell specific to the donor-derived cell. Then, the regulatory T cell is administered to a recipient before and after the transplantation to induce immune tolerance to a graft, whereby rejection reaction to the graft can be avoided and engraftment of the graft can be promoted. Accordingly, the immunomodulator of the present invention can avoid side effects caused by the existing immunosuppressants, bacterial or viral infection, particularly, recurrent hepatitis C after liver transplantation in hepatitis C patients.

In addition, in patients with an autoimmune disease, a patient-derived peripheral CD25⁺CD4⁺ T cell of a naive phenotype is cultured in the presence of a causative antigen of the autoimmune disease, whereby a regulatory T cell specific to the causative antigen is produced. By administration of the regulatory T cell to the patient, the autoimmune reaction can be suppressed.

Moreover, in patients with an allergic disease, a patient-derived peripheral CD25⁺CD4⁺ T cell of a naive phenotype is cultured in the presence of a causative antigen of the allergy, whereby a regulatory T cell specific to the causative antigen is produced. By administration of the regulatory T cell to the patient, the allergic reaction can be suppressed.

In an infertility patient, a patient-derived peripheral CD25⁺CD4⁺ T cell of a naive phenotype is cultured in the presence of a partner-derived cell, whereby a regulatory T cell specific to the partner-derived cell (or antigen) is produced. By administration of the regulatory T cell to the patient, the immune tolerance to the partner-derived cell (or antigen) or a fetus having the partner-derived antigen is induced, the rejection reaction to the fetus is avoided, and the maintenance of pregnancy can be facilitated.

Accordingly, the present invention provides an immunomodulator containing the regulatory T cell to be produced by the above-mentioned method as an active ingredient. The immunomodulator of the present invention can be used for the prophylaxis or treatment of the rejection of organ transplantation, allergic diseases (pollinosis, food allergy, drug allergy, asthma, atopic dermatitis, eczema, food hypersensitivity, urticaria, allergic rhinitis, allergic conjunctivitis), autoimmune diseases (polymyositis, chronic rheumatism, systemic lupus erythematosus, systemic sclerosis, bullous diseases, cutaneous lupus erythematosus, psoriasis, Crohn's disease, ulcerative colitis, autoimmune hepatitis, multiple sclerosis, type 1 diabetes etc.), graft-versus-host disease (GVHD), infertility and the like.

The immunomodulator of the present invention can be produced as an oral/parenteral preparation by mixing the effective amount of the above-mentioned regulatory T cell with a pharmaceutically acceptable carrier, and the like, according to a conventional method. The immunomodulator of the present invention is generally produced as a parenteral preparation such as injection, suspension, drip infusion and the like. As the carrier to be contained in the parenteral preparation, for example, an aqueous solution for injection such as physiological saline, isotonic solution containing glucose or other auxiliary agents (e.g., D-sorbitol, D-mannitol, sodium chloride and the like) and the like can be mentioned. The immunomodulator of the present invention may be blended with, for example, buffers (e.g., phosphate buffer, sodium acetate buffer), soothing agents (e.g., benzalkonium chloride, procaine hydrochloride and the like), stabilizers (e.g., human serum albumin, polyethylene glycol and the like), preservatives, antioxidants and the like.

The preparation obtained in this manner is safe and low toxic. Therefore, it can be administered, for example, to the aforementioned mammals such as human and the like.

While the dose of the regulatory T cell of the present invention varies depending on the subject of administration, target organ, symptom, administration method and the like, for example, in the case of parenteral administration to an adult patient (body weight 60 Kg), an effective amount with the upper limit being about 6 x 10⁹ a day is generally administered conveniently. For other animals, an amount converted based on the amount per 60 kg can be administered.

The present invention also provides a kit for producing a regulatory T cell, which comprises a reagent for providing a peripheral CD25⁺CD4⁺ T cell of a naive phenotype. As the reagent, various reagents (e.g., CD25, CD4, labeled antibody against marker antigen of a naive phenotype etc.) for providing a peripheral CD25⁺CD4⁺ T cell of a naive phenotype by the aforementioned method may be contained. The kit may further contain various reagents usable for the aforementioned methods (reagent for providing antigen, T cell growth factor, medium, reaction container, instruction sheet describing test protocol, and the like). Using the kit, a regulatory T cell can be conveniently produced according to the aforementioned method.

The present invention is explained in more detail in the following by referring to Examples, which are not to be construed as limitative.

### Examples

### [1] Material and method

### (cell preparation)

Venous blood was obtained from healthy volunteers of different ages between 0 and 69 years old. Subjects younger than 20 years old were followed at the Department of Pediatrics in Kyoto University Hospital because they have a mild congenital heart disease, and their parents' permission was obtained. Peripheral blood mononuclear cell (PBMC) was isolated by Ficoll-Hypaque (Amersham Biosciences-Uppsala) density gradient centrifugation. The cells were stained with allophycocyanin (APC)-conjugated anti-CD4 monoclonal antibody (mAb), phycoerythrin (PE)-conjugated anti-CD25 mAb, and fluorescein isothiocyanate (FITC)-conjugated anti-CD45RA mAb for 30 min at 4°C in the dark, after which the following six different cell fractions were isolated using a BD FACSAria cell sorter (Becton Dickinson)(see Fig.1).
(a) CD45RA⁺CD25⁺CD4⁺ T cell
(b) CD45RA⁻CD25^{high+}CD4⁺ T cell
(c) CD45RA⁻CD25^{low+}CD4⁺ T cell
(d) CD45RA⁻CD25⁻CD4⁺ T cell
(e) CD45RA⁺CD25⁻CD4⁺ T cell, and
(f) all-CD4⁺ T cell

The cell fractions shown in the aforementioned (a)-(e) were isolated according to the definition shown in Fig. 1c.

All of the mAbs were purchased from Becton Dickinson. The purity of each fraction was >98%. For cell separation to perform a functional assay, CD4⁺ cell after removing the cells bound to a plate were positively isolated using anti-CD4 mAb microbeads and magnetic cell sorting (MACS) system magnetic columns (Miltenyi Biotec GmbH). The purity of the CD4⁺ cell was >95%.

### (FACS analysis)

PBMC derived from the subjects, or proliferated T cell line were incubated with various antibodies for 30 min at 4°C in the dark, washed, and analyzed using a BD FACSAria cell sorter and a FACSDiVA software (Becton Dickinson). The antibodies used included perCP-conjugated anti-CD4 mAb, biotin-conjugated anti-CD25 mAb, FITC-conjugated anti-CD45RA mAb, PE-conjugated anti-CD45RB mAb, anti-CD45RO mAb, anti-CD62L mAb, anti-CD38 mAb, anti-GITR mAb, and anti-TNFRII mAb. For intracellular staining with CTLA-4, the cells were subjected to cell surface staining, fixation, and penetration treatment with Cytofix/Cytoperm solution (Becton Dickinson), and then intracellularly stained with PE-conjugated anti-CTLA-4.

All mAbs and streptavidin-APC were purchased from Beckton Dickinson, Immunotech, or Genzyme/Techne.

### (cell cultivation)

CD45RA⁺CD25⁺CD4⁺ or CD45RA⁺CD25⁻CD4⁺ T cell separated with a cell sorter as mentioned above (1 x 10^5 cells/well) were stimulated with irradiated (40 Gy) allogenic PBMC at 2 x 10^5 cells/well in growth medium (RPMI-1640 supplemented with 10% (V/V) FBS or human AB-type serum, HEPES, sodium pyruvate, L-glutamine, 2-ME, and antibiotics-antimycotics) in the presence of 200 U/ml human IL-2 in a 96-well plate at 130 µl/well. Half of the medium was replaced with fresh growth medium containing IL-2 every 3-4 days. Where necessary, the cells were separated, and restimulated with allogenic PBMC repeatedly every 7-10 days. When the number of cells increased sufficiently, the cells were recovered, and cultured (0.5 x 10^5 cells/well) in a 48-well plate together with irradiated allogenic PBMC (1 x 10^6 cells/well). Proliferated T cell line was washed three times, and then used for functional assays.

### (mixed lymphocyte reaction (MLR))

The indicated number of CD45RA⁻CD25^{high+}CD4⁺ T cell immediately after isolation, CD45RA⁺CD25⁺CD4⁺ T cell, or proliferated T cell line were each stimulated with 5 x 10^4 cells of isolated CD4⁺ T cell and 1 x 10^5 cells of irradiated (40 Gy) allogenic PBMC, and with or without 0.5 µg/ml of anti-human CD3 antibody (OKT-3), in growth medium containing 10% (V/V) FBS in a round-bottom 96-well microplate. For a neutralization assay, a neutralization antibody against IL-10R (5 µg/ml), TGF-β (0.5 µg/ml), CTLA-4 (5 µg/ml), or PD-1 (5 µg/ml) was added. For a transwell test, an equal number of, CD4⁺ T cell immediately after isolation and 1 x 10^6 T cell line were cocultured, or CD4⁺ T cell and T cell line were cultured separately with allogenic PBMC (3 x 10^6 cells) on the bottom and the upper side of a chamber, respectively. The cultures were incubated for 5 or 7 days (each with or without OKT-3), and labeled with [³H]-thymidine during the final 16 hr. The cultures were recovered, and [³H]-thymidine uptake was measured by 1450 Microbeta TriLux (Wallac).

### (quantitative RT-PCR)

Total RNA was extracted from the CD4⁺ T cell fraction using Isogen reagent (Nippon Gene) according to the instruction provided by the manufacturer, and incubated for 1 hr at 37°C in 20 µl of reaction solution containing 10 mM DTT, 0.5 mM dNTPs, 1 x M-MLV RT buffer, 40 ng of random primer p(dN)6, 6U of ribonuclease inhibitor, and 40U of M-MLV reverse transcriptase (Invitrogen), followed by heating for 10 min at 70°C, whereby cDNA was synthesized. The expression level of FOXP3 mRNA was quantified by real-time PCR using ABI/PRISM7700 sequence detection system (Applied Biosystems) and QuantiTect Probe PCR kit (Quiagen). FOXP3-specific primers and intrinsic fluorescent Taqman probe were designed as described earlier (see Int Immunol., 16, 1643-1656, 2004). A mixed solution of 20x primers and a probe for HPRT was purchased from Applied Biosystems. The expression level of FOXP3 mRNA was normalized against that of mRNA of a house-keeping gene HPRT. The data is expressed with average values from triplicate-wells. The standard deviation was <5%.

### [1] Results

### (ratio of CD45RA⁺CD25⁺CD4⁺ T cell gated by CD4 in different generations)

Fig. 1A shows representative FACS profiles of the CD45RA⁺CD25⁺CD4⁺ T cell fraction. In children younger than 20 years old, the CD45RA⁺CD25⁺CD4⁺ T cell fraction showed large and dense clusters (Fig. 1A; upper left and upper right), while in adults above 20 years old, it showed smaller and less dense clusters of the same cells as compared to those of children (Fig. 1A; lower left and lower right).

As Fig. 1B shows, the proportion of the CD45RA⁺CD25⁺ cell in CD4⁺ T cell was the highest in young children less than 5 years old, gradually decreased in an age-dependent manner, and was the lowest in adults beyond 60 years old. (CD45RA⁺CD25⁺CD4⁺ T cell shows high expression level of Foxp3 mRNA)

The expression levels of Foxp3 for the following six different cell fractions were measured.
(a) CD45RA⁺CD25⁺CD4⁺ T cell
(b) CD45RA⁻CD25^{high+}CD4⁺ T cell
(c) CD45RA⁻CD25^{low+}CD4⁺ T cell
(d) CD45RA⁻CD25⁻CD4⁺ T cell
(e) CD45RA⁺CD25⁻CD4⁺ T cell, and
(f) all-CD4⁺ T cell

These cells were isolated from PBMC according to the gate shown in Fig. 1C using a cell sorter. CD45RA⁻CD25^{high+}CD4⁺(b) and CD45RA⁺CD25⁺CD4⁺(a) T cell fraction showed higher expression levels of Foxp3 as compared to CD45RA⁻CD25^{low+}CD4⁺(c), CD45RA⁻ CD25⁻CD4⁺(d), CD45RA⁺CD25⁻CD4⁺(e), and all-CD4⁺(f) T cell fraction (Fig. 1D). Although CD45RA⁺CD25⁺CD4⁺ cell fraction had been considered to differ from Tregs, it showed a higher expression level of Foxp3 than even that of CD45RA⁻CD25^{high+}CD4⁺ cell fraction. Two or more tests were run for different individuals, and similar results were obtained. (CD45RA⁺CD25⁺CD4⁺ T cell is naive in phenotype, but shows expression of intracellular CTLR-4 and cell-surface TNFRII)

CD45RA⁺CD25⁺CD4⁺(a), CD45RA⁻CD25^{high+}CD4⁺(b), and CD45RA⁺CD25⁻CD4⁺(e) T cell (see Fig. 1C) were investigated as to naive/memory cell phenotype, and intracellular CTLR-4 and TNFRII (Fig.2). CD45RA⁻CD25^{high+}CD4⁺(b) T cell showed CD45RO⁺, CD45RB^{low+}, CD62L^{low+}, and CD38⁻. This suggests CD45RA⁻ CD25^{high+}CD4⁺ T cell is memory-type in phenotype. However, the opposite tendency was observed in CD45RA⁺CD25⁺CD4⁺(a) T cell as a whole. For example, CD45RA⁺CD25⁺CD4⁺ T cell were CD45RO^{low+}, CD45RB^{high+}, CD62L^{high+}, and CD38⁺. This suggests CD45RA⁺CD25⁺CD4⁺ T cell is naive in phenotype. These four naive/memory cell phenotypes of CD45RA⁺CD25⁺CD4⁺ T cell were equivalent to those of CD45RA⁺CD25⁻CD4⁺ T cell(e), which was a typical naive cell. CD45RA⁺CD25⁺CD4⁺ T cell was naive in phenotype, but this cell showed intracellular CTLR-4 and cell-surface TNFRII, which were specific to Tregs (which correspond to CD45RA⁻CD25^{high+}CD4⁺ cell). Unlike these two cell fractions, CD45RA⁺CD25⁻CD4⁺ cell, which is a typical naive cell, did not show intracellular CTLA-4 and cell-surface TNFRII. Two or more tests were run for different individuals, and similar results were obtained.

### (Functional property of separated CD45RA⁺CD25⁺CD4⁺ T cell)

The functional property of CD45RA⁺CD25⁺CD4⁺ T cell fraction at the time of isolation from PBMC was investigated in detail (Fig. 3). In the presence of allogenic PBMC and agonistic anti-CD3 antibody (Fig. 3A, lane 3, black column), separated CD45RA⁺CD25⁺CD4⁺ T cell were proliferative rather than non-responsive. On the other hand, CD45RA⁻CD25^{high+}CD4⁺ T cell was clearly non-responsive at the time of isolation of the fraction (Fig. 3A, lane 3, white column).

Furthermore, CD45RA⁺CD25⁺CD4⁺ T cell hardly suppressed proliferation of syngeneic CD4⁺ T cell which was cocultured at a ratio of 1:1 in the presence of an alloantigen (Fig. 3A, lane 4, black column). On the other hand, CD45RA⁻CD25^{high+}CD4⁺ T cell markedly suppressed the proliferation of all-CD4⁺ T cell as compared to MLR with all-CD4⁺ T cell alone (Fig. 3A, lane 2) to show immunosuppressive ability (Fig. 3A, lane 4, white column).

A similar test was conducted under the conditions free of agonistic anti-CD3 antibody (Fig. 3B). In the absence of an agonistic anti-CD3 antibody, both CD45RA⁺CD25⁺CD4⁺ T cell and CD45RA⁻CD25^{high+}CD4⁺ T cell were non-responsive to alloPBMC (Fig. 3B, the 3rd lane). Similar to the presence of anti-CD3 antibody, CD45RA⁻CD25^{high+}CD4⁺ T cell suppressed proliferation of CD4⁺ T cell (Fig. 3B, 4th-7th lanes, white columns), but CD45RA⁺CD25⁺CD4⁺ T cell slightly suppressed the proliferation (Fig. 3B, 4th-7th lanes, black column) as compared to MLR with CD4⁺ T cell alone (Fig. 3B, 2nd lane),. (Ability of ex vivo CD45RA⁺CD25⁺CD4⁺ T cell to proliferate by repetitive stimuli with allogenic PBMC in the presence of IL-2)

CD45RA⁺CD25⁺CD4⁺ T cell was proliferated not less than 100-fold in 40 days by repetitive stimulation with allogenic PBMC in the presence of IL-2 in growth medium containing 10% (V/V) FBS. The number of cells increased about 100-fold in 80 days by cultivation of this fraction using growth medium containing 10% (V/V) human serum (Fig. 4A).

### (Functional property of proliferated CD45RA⁺CD25⁺CD4⁺ T cell line)

Unlike CD45RA⁺CD25⁺CD4⁺ T cell per se, the proliferated cell line obtained from the CD45RA⁺CD25⁺CD4⁺ T cell fraction (to be referred to as CD45RA⁺CD25⁺CD4⁺ T cell line) showed non-responsiveness and suppressive property. The proliferated cell line suppressed proliferation of syngeneic all-CD4⁺ T cell stimulated with allogenic PBMC used for the proliferative cultivation, in a dose-dependent manner (Fig. 4B).

In contrast, when syngeneic all-CD4⁺ T cell were stimulated with allogenic PBMC different from the allogenic PBMC used for establishing the CD45RA⁺CD25⁺CD4⁺ T cell line (outsider PBMC), proliferation thereof was hardly suppressed by the CD45RA⁺CD25⁺CD4⁺ T cell line (Fig. 4C). This suggests that the immunosuppressive ability of CD45RA⁺CD25⁺CD4⁺ T cell line is antigen-specific.

Moreover, syngeneic all-CD4⁺ T cell was cocultured with the CD45RA⁺CD25⁺CD4⁺ T cell line at a ratio of 5:1 in the presence of allogenic PBMC (which was used for the proliferative cultivation), and an antibody against IL-10R, TGF-β, CTLA-4, or PD-1 was added to the coculture. However, none of the antibodies cancelled the immunosuppressive ability of CD45RA⁺CD25⁺CD4⁺ T cell line (Fig. 4D).

During the coculture of CD45RA⁺CD25⁺CD4⁺ T cell line and syngeneic all-CD4⁺ T cell with allogenic PBMC, contact of CD45RA⁺CD25⁺CD4⁺ T cell line with syngeneic all-CD4⁺ T cells was prevented using a transwell chamber system. Irrespective of whether the allogenic PBMC was placed with CD45RA⁺CD25⁺CD4⁺ T cell line, the immunosuppressive ability of CD45RA⁺CD25⁺CD4⁺ T cell line was lost by the cell-cell contact avoidance (Fig. 4E). This result suggests suppressive property of CD45RA⁺CD25⁺CD4⁺ T cell line requires cell-cell contact. (phenotype analysis of proliferated CD45RA⁺CD25⁻CD4⁺ T cell line and proliferated CD45RA⁺CD25⁺CD4⁺ T cell line)

Phenotype was analyzed for the proliferated cell line originated from CD45RA⁺CD25⁻CD4⁺ T cell (which is typical naive cell) fraction or CD45RA⁺CD25⁺CD4⁺ T cell fraction (Fig. 5). The intensity of CD25, GITR, and CTLA-4 on CD45RA⁺CD25⁺CD4⁺ T cell line was clearly higher than that on CD45RA⁺CD25⁻CD4⁺ T cell line. The intensity of CD62L on CD45RA⁺CD25⁺CD4⁺ T cell was also substantially high. This forms a contrast with the absence of CD62L on CD45RA⁺CD25⁻CD4⁺ T cell. Accordingly, it was suggested that the phenotype property of CD45RA⁺CD25⁺CD4⁺ T cell line was similar to that of Tregs, but different from that of naive T cells.

While the expression level of CD45RO in CD45RA⁺CD25⁺CD4⁺ T cell line increased to approximately 6.7-fold as compared to that in peripheral CD45RA⁺CD25⁺CD4⁺ T cell before cultivation, the expression level was lower as compared to that of Tregs (CD45RA⁻CD25^{high+}CD4⁺ T cells) inartificially present in peripheral blood and was approximately 0.2-fold thereof. In addition, the expression level of CD45RB in CD45RA⁺CD25⁺CD4⁺ T cell line was higher as compared to that of Tregs (CD45RA⁻ CD25^{high+}CD4⁺ T cell) inartificially present in the periphery and was approximately 3.6-fold thereof. (The comparisons of expression levels were performed by comparing the expression intensities at peaks of the histograms in the graphs with the cell number on the vertical axis and the expression intensity (fluorescence intensity) on the transverse axis.)

Accordingly, it has been suggested that CD45RA⁺CD25⁺CD4⁺ T cell line satisfies functional requirements of regulatory T cell, but is different in phenotype from Treg inartificially present in peripheral blood.

### Industrial Applicability

Using the method of the present invention, a mammalian regulatory T cell can be efficiently produced in vitro, and an antigen-specific regulatory T cell can also be produced. The regulatory T cell produced by the method is useful as an immunomodulator for the prophylaxis or treatment of rejection reaction in organ transplantation, allergic disease, autoimmune disease, graft-versus-host disease (GVHD), infertility and the like.

This application is based on a patent application No. 2005-106887 filed in Japan (filing date: April 1, 2005), the contents of which are incorporated in full herein by this reference.

## Claims

1. A method of producing a regulatory T cell, which comprises culturing a peripheral CD25⁺CD4⁺ T cell of a naive phenotype to give the regulatory T cell.

2. The method of claim 1, wherein the naive phenotype is at least one phenotype selected from the group consisting of CD45RA⁺, CD45RO^{low+}, CD45RB^{high+}, CD62L^{high+} and CD38⁺.

3. The method of claim 1, wherein the CD25⁺CD4⁺ T cell has at least one phenotype selected from the group consisting of Foxp3⁺, intracellular CTLA-4⁺ and cell surface TNFRII⁺.

4. The method of claim 1, wherein the CD25⁺CD4⁺ T cell is derived from primate.

5. The method of claim 1, wherein the CD25⁺CD4⁺ T cell is cultured in the presence of an antigen.

6. The method of claim 5, wherein the antigen is an immunologically non-autologous cell.

7. The method of claim 6, wherein the immunologically non-autologous cell is an allogenic cell.

8. The method of claim 5, wherein the obtained regulatory T cell is specific for the antigen.

9. The method of claim 1, wherein the CD25⁺CD4⁺ T cell is cultured in the presence of a T cell growth factor.

10. The method of claim 1, wherein the regulatory T cell has at least one phenotype selected from the group consisting of CD45RO^{mid+,} CD45RB^{high+}, CD62L^{low+} and GITR⁺.

11. A regulatory T cell produced by the method of any one of claims 1 to 10.

12. A CD25⁺CD4⁺ regulatory T cell having at least one phenotype selected from the group consisting of CD45RO^{mid+,} CD45RB^{high+}, CD62L^{low+} and GITR⁺.

13. An immunomodulator comprising the regulatory T cell of claim 11 or 12 as an active ingredient.

14. The agent of claim 13, which is used for the introduction of immune tolerance.

15. A method of producing an immunomodulator, which comprises
(1) a step of culturing a peripheral CD25⁺CD4⁺ T cell of a naive phenotype to give a regulatory T cell;
(2) a step of mixing the regulatory T cell with a pharmaceutically acceptable carrier to give the immunomodulator.

16. A kit for the production of a regulatory T cell, which comprises a reagent for providing a peripheral CD25⁺CD4⁺ T cell of a naive phenotype.

17. Use of the regulatory T cell of claim 11 or 12 for the production of an immunomodulator.

18. The use of claim 17, wherein the immunomodulator is an immune tolerance-inducing agent.

19. A method of regulating immunity, which uses the regulatory T cell of claim 11 or 12.

20. The method of claim 19, which is a method of inducing immune tolerance.

21. A commercial package comprising an immunomodulator composition comprising the regulatory T cell of claim 11 or 12 and a pharmaceutically acceptable carrier, and a written matter stating that the composition can or should be used for immune regulation.

22. The package of claim 21, wherein the immune regulation is induction of immune tolerance.
